Europäisches Patentamt

European Patent Office

Office européen des brevets

(19)

(11) Publication number: **0 132 285**
**A2**

# (12) EUROPEAN PATENT APPLICATION

(21) Application number: **84303969.4**

(22) Date of filing: **12.06.84**

(51) Int. Cl.⁴: **G 01 N 33/52**

(30) Priority: **25.06.83 GB 8317313**

(43) Date of publication of application: **30.01.85**
**Bulletin 85/5**

(84) Designated Contracting States: **AT BE CH DE FR GB IT LI LU NL SE**

(71) Applicant: **The University Court of the University of Glasgow, The University, Glasgow G12 8QQ Scotland (GB)**

(72) Inventor: **Goudie, Robert Barclay, 33 Manse Road Bearsden, Glasgow G61 3PW Scotland (GB)**

(74) Representative: **McCallum, William Potter et al, Cruikshank & Fairweather 19 Royal Exchange Square, Glasgow G1 3AE Scotland (GB)**

(54) Medical and chemical testing kit.

(57) The invention is a medical and chemical testing kit comprising a plurality of elongated reagent-containing elements (1, 9, 33, 38, 39). Means (2, 27B, 28B, 29B and 41) are provided for sealing off a predetermined quantity of reagent within each element whereby a predetermined quantity of reagent can be detached from its associated element (1, 9, 33, 38, 39) to permit expulsion of the reagent contained in said detached portion.

0132285

# MEDICAL AND CHEMICAL TESTING KIT

The subject of this invention is a kit for use in performing medical tests or chemical tests requiring the consecutive application of different reagents.

In medical and chemical technology there are many standard tests used for the purpose of providing a ready means of ascertaining if a particular substance is or is not present in a sample of tissue or other material for identifying the condition of such a sample. These often take the form of applying several specific reagents in a predetermined series and noting whether or not certain changes occur. Heretofore the reagents have been contained in bottles which may be grouped in a container providing a kit for performing each test and when a test is to be conducted the amount of liquid to be withdrawn is removed from each bottle usually by an instrument such as a pipette. In view of the very large variety of such tests, any one of which may be required to be performed at any time in a hospital for example, a large storage space is required for the reagents or for any such kits, especially since previously proposed kits tend to be bulky. Furthermore, the introduction of a pipette into the same bottle a large number of times in the performance of successive tests almost invariably causes contamination of the contents of the bottle. The use of a dropping bottle does not avoid contamination because air must enter the bottle to replace the liquid removed. Even although performance of the test with a contaminated reagent may not constitute a health hazard, contamination of the reagent may impair the validity of the test. There are other disadvantages associated with previously proposed kits. Previously proposed kits require, for convenience of handling, bottles of a minimum volume and individual labeling of the bottles. This labeling operation is labour-intensive and can be a source of error.    It also adds considerably to the cost of the kits. Also many of the reagents used in tests are expensive and have a restricted storage life which may even be considerably reduced as a result of the contamination referred to above.    Because the bottles have a minimum volume it frequently happens that quantities of expensive reagents have to be discarded because they have exceeded their storage life.

An object of the present invention is to provide a kit for use in performing medical or chemical tests in which the aforesaid disadvantages of previously proposed testing kits are obviated or mitigated.

According to the present invention there is provided a test kit for performing medical or chemical tests comprising a plurality of continuous elongated reagent-containing elements, each element containing a particular reagent, and means capable of defining a predetermined quantity of each reagent at spaced intervals along the length of the elements whereby each predetermined quantity of reagent can be selectively removed from its associated element in order to permit application thereof in a particular test.

The number of reagent-containing elements contained in the kit will be dictated by the number and volume of reagents required for a particular test or a specific part of a test or a set of tests or specific parts of the tests of a set.

Conveniently, the plurality of elements is secured to a common anchorage constituted by, for example, a pin, a ring, a card or a piece of sheet material which may be synthetic plastics material, to which the elements are attached, or a series of reels or spools having a common mounting or axis, each element being wound around an individual reel or spool.    The elements may be attached to the common anchorage in the order in which the reagents are to be used.    Where the common anchorage is constituted by a card or a piece of sheet material it may be formed with grooves or recesses in which the elements lie, thereby protecting the elements and reducing any risk that the elements may become inconveniently entangled.

A tab or label containing at least the information as to which element is first in the order of reagents to be applied or identifying each element and the order in which its contents are to be used in the test, may be attached to the anchorage, the tab or label also detailing the test for which that particular kit is intended to be used. The tab or label may also list the reagents contained in the elements. Where the common anchorage is a card or a piece of sheet material, any desirable information about the reagents in the kit and/or the test for which the kit is usable may be applied to the card or piece of sheet material.

The elements may be colour-coded by means of solid colouring or by coloured bands or dots, and the tab or label may then carry a colour marking opposite each reagent listed. Alternatively, the elements may be transparent and the reagents may themselves be coloured, where this is not objectionable.

The elements may be closed at the ends and may be closeable at intermediate points by being knotted or otherwise sealed, e.g. by pinching. Alternatively, where the application of heat is not objectionable, they may be pinched and heat sealed, or sealed by ultrasonic or high frequency welding techniques.

Suitable materials for the elements are polyvinylchloride and polyethylene. The elements should be flexible and sufficiently pliable to enable the reagent to be readily expelled. Tubular elements with an internal diameter of 2.0 mm are particularly suitable for delivering approximately 0.1 ml volumes of reagent, the tubular elements being pinched or sealed at intervals of approximately 32 mm.

Where the common anchorage is a pin or a ring, each element may be attached to the pin or the ring by simply knotting it around the pin or the ring. Alternatively the set of elements required for one particular test may be connected directly to an identifying tab by knotting at least one of the elements through a hole in the tab, the other elements being then knotted together at that end as well as being individually knotted to act as a seal.   The tab then functions also as the anchorage.   Where the common anchorage is a card or a piece of sheet material e.g. a sheet of plastics material, one end of each element may be attached to the common anchorage, e.g. by gluing or heat sealing or by ultrasonic or high frequency welding or by adhesive tape, leaving the other end of the element free.

To avoid the difficulty of providing a large number of closely resembling colours where a large number of reagents is required for a particular test, there may be provided a recurring sequence of say three readily identifiable colours, for example, white, red, black. While this will mean several different reagents being in the elements of the same colour, the elements of the same colour will be separated by tubes of other colours thus reducing considerably the possibility of making a mistake in counting the number of elements from the tab or label as the tab or label will indicate the same recurring sequence of colours as the elements.   In other words, the element selected at each part of the test must agree in colour with the colour indication of that reagent on the tab or label.

For certain purposes it may be desirable to use with the kit at least one substance which does not require to be held in specific small aliquots or under special conditions of sterility.   Such a substance might be a disinfectant.   It may then be convenient to include in the kit a larger bottle of that substance held in a conventional fashion, for example held by frictional grip in a container linked to the common anchorage.

Embodiments of the present invention will now be described, by way of example, with reference to the accompanying drawings in which:-

Fig. 1 illustrates a first embodiment of test kit for use when only three reagents are required.

Fig. 2 illustrates a second embodiment of the invention illustrating a test kit for use when a larger number of reagents require to be used.

Fig. 3 illustrates a third embodiment of test kit containing several groups of elements each having a common anchorage provided by a sheet mounted for convenience in a ring binding.

Fig. 4 illustrates a fourth embodiment of test kit requiring the use of three elements and in which a common anchorage is provided by a series of spools having a common mounting or axis.

Figs. 5 and 6 illustrate plan and side views respectively of a fifth embodiment of test kit in accordance with the invention in which each element is in the form of a continuous strip having reagent compartments on one side thereof, and

Figs. 7 and 8 illustrate plan and side views respectively of a sixth embodiment of test kit in accordance with the invention in which each element is in the form of a continuous strip having reagent compartments on both sides thereof.

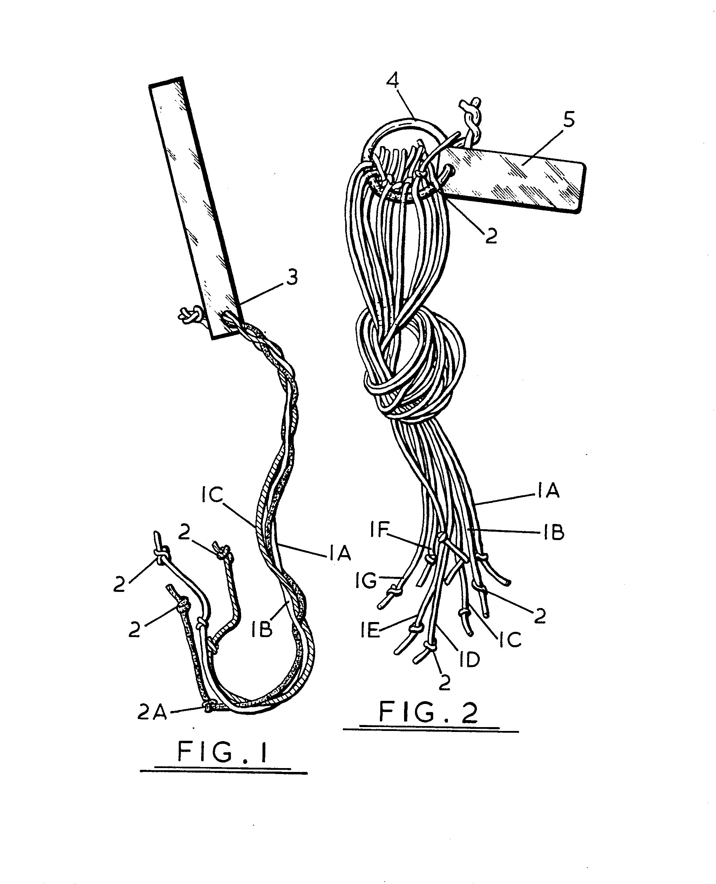

In the drawings and referring first to Fig. 1, 1A, 1B and 1C denote flexible tubular elements containing the different reagents specific to a particular test, each element being knotted at 2 at one end and being attached at the other end to a tab 3 marked with information about the test for which this kit may be used and containing an indication of the contents of the individual elements, the indication being by means of the colours of the elements and the tab indicating the order in which the elements of each colour are to be used. The elements are attached

to the tab by one of their number being passed through a hole in the tab and then being knotted to the rest of the elements which are first knotted to close them off, the elements being then plaited for convenience.

Referring to Fig. 2, 1A, 1B, 1C, 1D etc. indicate different tubular elements again containing different reagents but in a larger number, the elements also being knotted as at 2 at both ends and being passed around and knotted to a ring 4, in the order in which they will be used. A tab 5 is attached to the ring, the tab carrying the name of the test and indicia showing the contents of the various tubular elements in the order in which they occur starting from the tab.

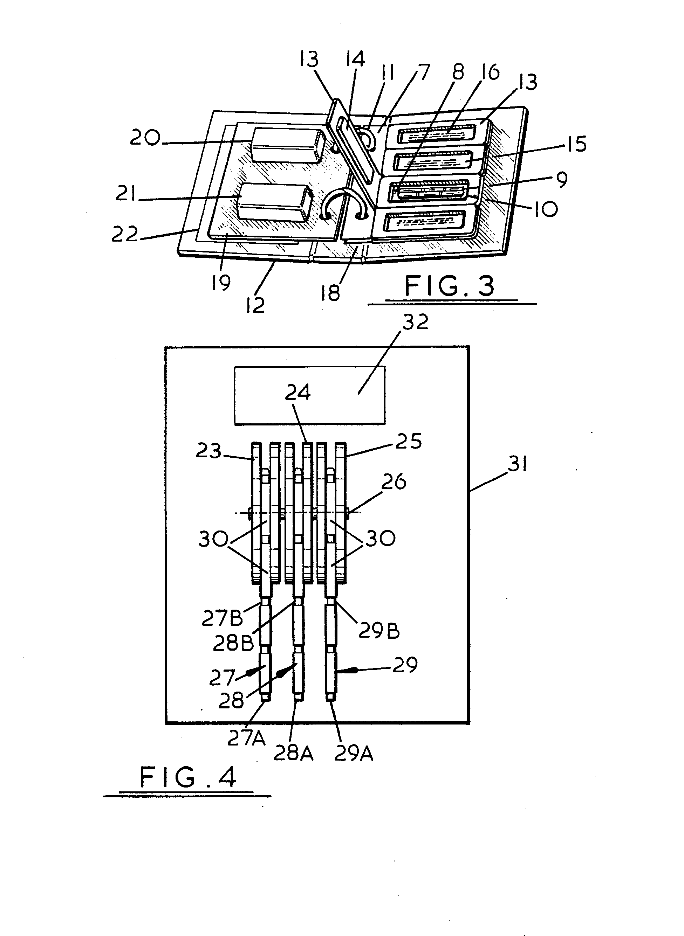

Referring to Fig. 3, a sheet 7 of synthetic plastics material has connected thereto at one end 8 of individual tubular elements 9 of the kit, each of said tubular elements being contained in and attached e.g. glued, at one end into a depression 10 in the sheet 7, which for convenience is held on openable rings 11, forming part of a ring binder 12. A flap 13 also attached to the sheet 7 is arranged to swing with respect to the sheet 7, the flap 13 being formed with an integral projection 14 engageable with the depression 10 in the sheet 7 so that, when the flap 13 is pressed into engagement with the sheet 7, the projection 14 fits into and remains in engagement with the depression 10 by frictional grip. The projection 14, being pressed out of the material of the flap 13, causes a depression 15 to be present on the other surface of the flap 13. This depression contains information as indicated at 16 relating to the tubular element contained within the depression 10. In the particular embodiment of Fig. 3 there are four tubular elements 9 contained in the group of elements attached to the sheet 7. The test kit of Fig. 3 also contains a second group of tubular elements (not shown) connected to a second sheet 18 which in the illustration is mainly covered over by the upper group.

The test kit of Fig. 3 is intended for a purpose which requires three reagents to be applied in succession. The first and third of these reagents is each a common reagent contained in a single bottle and these bottles are contained in depressions in a sheet 19 of synthetic plastics material also attached to the rings 11 of the ring binder 12. Projections 20 and 21 are formed by the bottle-containing depressions on the underface as illustrated in Fig. 3 of the sheet 19, each of these depressions containing a bottle containing the first and third reagents. A label 22 giving information of the test for which the kit is to be used is attached to the inner surface of the ring binder 12 which in the illustration is mainly covered by the sheet 19.

More particularly, the physical form of the kit of Fig. 3 is suitable for use as an immunoperoxidase staining test kit for the examination of samples of diseased tissue for diagnostic purposes.

In practical terms a test kit as illustrated prepared for this purpose may contain in a bottle contained in the compartment formed by the projection 20 swine serum to be applied as the first layer (layer 1) on all tissue samples to be tested. Each tubular element 9 is formed with segments each containing an aliquot of a particular reagent which may be selected to be applied as a second layer (layer 2) on a particular tissue sample. Such reagents may be Rabbit anti-human IgD, Rabbit anti-human Kappa light chain, Rabbit anti-human Lambda light chain, Rabbit anti-human Muramidase ... and contained in a bottle occupying the compartment formed by the depression 17, Swine anti-Rabbit I gG horseradish peroxidase conjugated to be applied as the third layer (layer 3) on all tissue samples to be tested.

The test or rather the set of tests is performed by treating a tissue sample with the layer 1 reagent, then treating it with one of the layer 2 reagents contained in a selected tubular element 9 and then with the layer 3 reagent.    Access to a selected tubular element 9 is obtained by pulling the appropriate flap 13 back thus exposing the element containing the desired reagent whereupon the element may be pulled out from the depression 10 containing it and the desired quantity of reagent expelled from the tubular element in one of the manners described.

In Fig. 4, 23, 24, and 25 denote three spools arranged in a block, the spools having a common axis of rotation 26.    Around each spool is wound tubular elements 27, 28 and 29, each tubular element being sealed at each end (the free ends only being shown at 27A, 28A and 29A and at intermediate points 27B, 28B, 29B to give segments 30 each containing the same volume of reagent, the inner end of each tubular element (not shown) being attached to the spool around which it is wound, the block of spools 23, 24, 25 serving as a common anchorage for the tubular elements 27, 28, 29 respectively.    The block of spools is attached to a mounting 31 to which there is attached a panel or an adhesive label 32 carrying printed instructions identifying the test, the reagents being identified by colour-coding and/or bearing a printed identification corresponding to the instructions given at 32.    The tubular elements may be unrolled from the spools as and when required for use.    If desired a cover (not shown) with three orifices may be fitted over the block of spools, the tubular elements passing out through the aforesaid orifices.

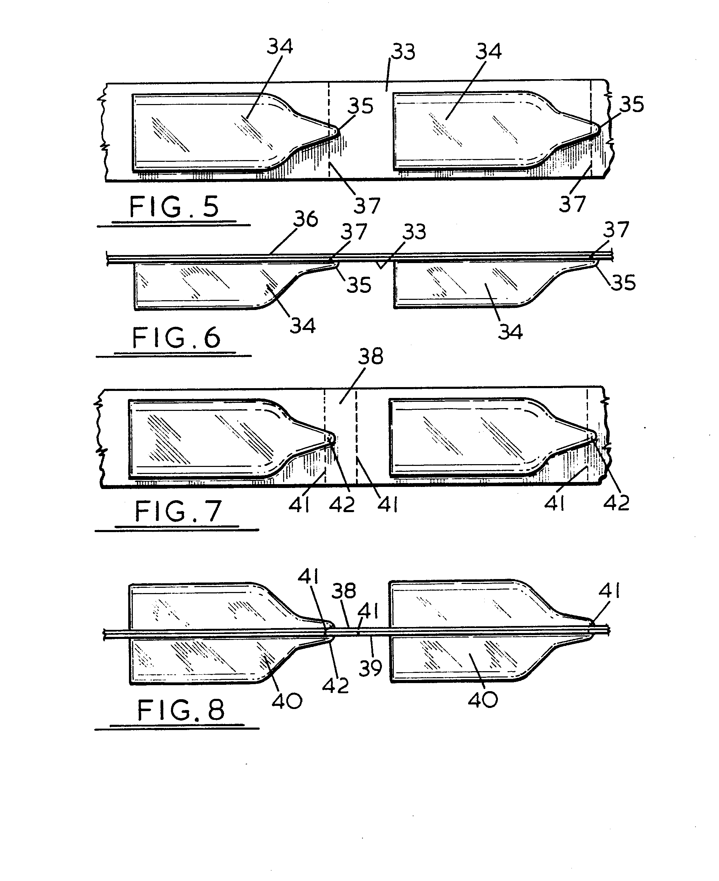

Referring to Figs. 5 to 8, there is illustrated further alternative embodiments of the invention in which each continuous element has a plurality of segments constituted by reagent-containing compartments preformed or moulded from a flat strip or ribbon of inert synthetic plastics material.

The continuous element, as shown in Figs. 5 and 6, consists of a flat ribbon 33 of synthetic plastics material

in which a plurality of depressions defining a series of spaced compartments 34 of predetermined volume are preformed along the length of the ribbon. Each compartment 34 is formed with a narrow neck portion 35. A sealing layer 36 is bonded across the upper face of the ribbon 33 in order to close off the compartments 34 once they have been filled with a reagent. Lines of weakening 37 e.g. a line of perforations, extend across the ribbon 33 and it will be noted that these lines of weakening extend across each free end of the narrow neck portion 35 of each compartment 34. Accordingly, a single compartment 34 can be severed from the end of the continuous element along a line of weakening 37. Such severance also fractures the neck portion 35 of the compartment 34 to allow the reagent contained therein to be dispensed as required.

In Figs. 7 and 8, an arrangement is shown similar to that illustrated in Figs. 5 and 6 but wherein the continuous element is formed by a pair of continuous flat ribbons 38, 39 of synthetic plastics material each having preformed reagent-containing compartments 40 provided on one side thereof. The two ribbons 38, 39 are passed in abutting relationship to a sealing and filling head (not shown) where they are sealed together along their length and as they are sealed together, the compartment 40 thus formed is filled with a reagent. As in the arrangement of Figs. 5 and 6, lines of weakening 41 extend across the continuous element, each line of weakening intersecting a narrow neck portion 42 of each compartment 40.

In practice, in use of the invention, as exemplified by the aforesaid embodiments, where the tubular elements are not presegmented as in Figs. 1 and 2, a test is performed by choosing a particular length of the tubular elements containing the reagent to be applied first, tying a new knot in the tubular element (e.g. 2A in Fig. 1) or otherwise sealing the tubular element at an intermediate point in its length to isolate the quantity of
reagent required, then cutting off the end of the tubular
element below the knot or seal and expelling the reagent
from the cut portion of element.   When the element is
segmented as illustrated in Figs. 3 and 4, the segment
at the free end of the tubular element is separted by
cutting across the pinched section forming a seal between
that segment and the next segment, without perforating the
seal of the next segment, opening the segment which has
been detached and expelling the reagent from the detached
portion of tubular element.   This practice is followed for
each of the elements in sequence as the test proceeds, the
cut-off portions of tubular element being simply discarded.

Use of the embodiments illustrated in Figs. 5 to 8
is similar to that described above in relation to Figs. 3
and 4, except that when one segment is separted from its
adjacent segment, the separated segment is opened by
fracturing the segment along its line of weakness to break
the neck of the reagent-containing compartment where the
line of weakness extends thereacross.

As an alternative to this procedure, one segment can
be separated from an adjacent segment by cutting, or otherwise severing e.g. across a first line of weakness,
across the interconnecting strip or ribbon at a location
between and spaced from the adjacent segments so that the
neck of one segment is not broken by this operation.   The
neck of a removed segment can be subsequently broken e.g.
by fracturing the segment along a separate line of weakness
extending across the neck in order to enable expulsion of
the reagent contained therein. (see Figs. 7 and 8).

A test kit of the invention is of small bulk since no
bottles are required, or the number of bottles is substantially reduced, each kit is easily stored.   For example,
each kit on a ring may be suspended from a hook and each
kit in which the common anchorage is a card or similar
body, may be stacked or stored in a box and also there is no contamination of the reagent when removing a sample because the newly formed knot or seal in an unsegmented tubular element or the remaining portion of the seal between individual segments of a segmented tubular element completely isolates the reagent still remaining in the element after the quantity of reagent required has been removed.

When all the reagents in a kit have been used the remainder of the kit may be incinerated, there being no bottles to dispose of and no pipettes or other measuring devices to be washed and dried and sterilized.

The tubular elements are easily filled by use of syringes or by suction.

The small space taken up by a kit can be seen particularly in Fig. 2 of the drawings where the tubes are shown coiled and knotted to one another. A distortion of the tubular elements in this fashion does not impair in any way their use in performing the required tests.

It is to be understood that the term 'reagent' as used in the present specification is used in a broad sense to include not only substances which react chemically or biologically with other substances in the test, e.g. with the test materials, but also other substances, e.g. buffer solutions, chromogenic substrates, counterstains and other reagents required for effecting the test, which must be added during the course of the test.

0132285

CLAIMS:

1. A test kit for performing medical or chemical tests characterised in that there is provided a plurality of continuous elongated reagent-containing elements (1,9,27,28,29,33,38,39), each element containing a particular reagent, and means (2,30,40) capable of defining a predetermined quantity of each reagent at spaced intervals along the length of the elements whereby each predetermined quantity of reagent can be selectively detached from its associated element in order to permit application thereof in a particular test.

2. A test kit as claimed in claim 1, in which each of the plurality of elements (1,9,27,28,29) are attached at one end thereof to a common anchorage (3,4,7,26).

3. A test kit as claimed in claim 1 or 2, in which means (3,5,22,32) are provided for identifying the test for which the kit is intended and for identifying the reagent contained in each element.

4. A test kit as claimed in claim 3, in which the means for identifying the reagent contained in each element contains information as to the order in which the reagent contained in each of the elements is to be applied, in order to allow a particular test to be carried out.

5. A test kit as claimed in any preceding claim, in which each element (1,9,27,28,29) is in the form of a continuous flexible tube.

6. A test kit as claimed in claim 5, in which the means capable of defining a predetermined quantity of reagent comprises knot means (2) formed at an appropriate location in order to provide a segment of tube containing said predetermined quantity.

7.   A test kit as claimed in claim 5, in which the tube (27,28,29) is of synthetic plastics material and the tube is sealed at predetermined spaced intervals to provide a  series of spaced segments (30), each segment containing a  predetermined quantity of reagent.

8.   A test kit as claimed in any preceding claim, in which each of the elements is colour-coded.

9.   A test kit as claimed in any preceding claim in which each element (9) is mounted in a sheet (7) of material within a compartment (10) formed in said sheet (7) and a closure member (13) is co-operable with said sheet (7) and is adapted to close an associated compartment, each closure member (13) being provided with test information to facilitate the reagent contained in a particular element (9) to be correctly applied in a test.

10.   A test kit as claimed in claim 9, in which the sheet (7) of material and the closure member or members (13) are operatively mounted on a ring binder (12).

11.   A test kit as claimed in any of claims 1 to 8, in which each continuous element (27,28,29) is mounted on a rotatable spool (23,24,25).

12.   A test kit as claimed in claim 11, in which each of the spools (23,24,25) are mounted for rotation about a common axis (26).

13.   A test kit as claimed in claim 11 or 12, in which information regarding the sequence of application of the reagents is carried on a support (31) on which the spools (23,24,25) are mounted.

0132285

14. A test kit as claimed in any preceding claim, in which each element comprises a continuous flat ribbon (33,38,39) of synthetic plastics material having preformed reagent-containing sealed compartments (34,40) at predetermined spaced intervals along its length, and whereby one compartment containing a predetermined quantity of reagent can be severed from the end of the element.

15. A test kit as claimed in claim 14, in which lines of weakening (41) extend across the ribbon (33,38,39) to facilitate severance of each compartment from the element.

16. A test kit as claimed in claim 15, in which each line of weakening (41) extends across a portion (42) of each compartment (34,40) whereby severance of the compartment from its continuous element also effects opening of said compartment.

17. A test kit as claimed in any of claims 14 to 16, in which each element consists of a pair of flat ribbons (38,39) having spaced compartments (40) preformed therein and sealed together along their length to provide said reagent-containing sealed compartments (40) extending on each side of said abutting flat ribbons (38,39).

FIG.1

FIG.2

0132285

FIG. 3

FIG. 4

0132285

FIG.5

FIG.6

FIG.7

FIG.8